# EUROPEAN PATENT APPLICATION

(11) **EP 3 929 274 A1**
(43) Date of publication of application: **29.12.2021**
(21) Application number: 20181703.8
(22) Date of filing: 23.06.2020
(51) Int. Cl.: C12M 1/00, C12M 1/26

(54) **METHOD AND APPARATUS FOR CULTIVATING MICROALGAE**

(71) Applicant: National University of Ireland, Galway, H91 TK33 Galway (IE)
(72) Inventor: The designation of the inventor has not yet been filed
(74) Representative: Appleyard Lees IP LLP

(57) **Abstract**

1. A method for cultivating microalgae, the method comprising the steps of:
(a) providing a photoreactor;
(b) introducing a mixture of microalgae into the photoreactor;
(c) introducing a culture medium into the photoreactor;
(d) growing the microalgae in the culture medium in the photoreactor;
(e) separating the microalgae into a floating layer and a non-floating layer within the culture medium;
(f) optionally harvesting a portion of the microalgae in the floating layer; and
(g) discharging at least a portion of the non-floating layer from the photoreactor.

## Description

### Field

The present invention relates to a method of cultivating microalgae, an apparatus for carrying out such a method, microalgae cultivated in this way, the use of such microalgae to produce a bioproduct, a method of producing a bioproduct from such microalgae, and a bioproduct produced in this way. In particular, the present invention relates to a method of selectively cultivating auto-floating microalgae from a mixture of microalgae, suitably a mixture of naturally occurring microalgae.

### Background

Microalgae are a promising source of renewable biofuels and bio-based chemicals. Compared with conventional biofuel and biorefinery feedstocks, such as energy crops, microalgae may be attractive due to superior productivity and less competition for land. However, the microalgae biorefinery industry is currently not economically viable due to several technical challenges in large scale microalgae production, which include strain selection, nutrient supply and recycling, gas transfer and exchange, light delivery, environment control, land and water availability, and harvesting.

Cost-effective harvesting of microalgae (i.e. microalgae biomass) is particularly problematic and faces challenges arising from (1) the low microalgae concentrations in common cultivation systems; (2) the small size of microalgal cells; (3) the similarity of density of the algal cells to the growth medium; and (4) the suspension stability of microalgal cells resulting from their negative surface charges. It is estimated that the cost of harvesting contributes to 20 to 40% of the total cost of microalgal biomass production. For some high added value products, the harvesting cost could even be as high as 60% of the total cost.

Various methods have been suggested for harvesting microalgae on a large-scale. These methods include centrifugation, conventional filtration, membrane filtration, flocculation, sedimentation and air flotation. Centrifugation is the most rapid and reliable method for recovering suspended algae, but this method is cost-prohibitive for large scale use due to the high investment and operating costs. Conventional mechanical filtration has been found satisfactory at harvesting relatively large, filamentous microalgal strains but has low throughput and rapid clogging. Furthermore, it is not valid for small and non-filamentous strains which are commonly present in microalgae cultivation systems. Membrane filtration has been recently investigated as an efficient and reliable harvesting method, but membrane fouling and replacement lead to significant costs, in addition to high power requirements. The most accepted method is flocculation of algal cells followed by separation of flocs from the liquid by either sedimentation or air flotation. Air flotation is carried out in the presence of tiny gas bubbles. It is generally considered more advantageous than sedimentation because it can be carried out at relatively high overflow rates while still attaining favourable separation efficiencies and a well-compacted product. The latter characteristic is important for reducing the cost and energy consumption in further processing steps, such as dewatering and drying. However, the mechanical systems used to provide flotation gas are complex, costly and energy intensive.

Alternatively, dissolved oxygen (DO) which originates from the photosynthetic activity of microalgae is known as a source of flotation gas. Under oxygen supersaturated conditions, some microalgae can float on top of flocculated water after being treated by certain types of flocculants, such as aluminium sulphate. The flotation efficiency is highly dependent on physicochemical variables such as DO concentration, flocculant (type, dosage and contact time), mixing intensity and total retention time. Although the utilisation of photosynthetic oxygen represents a considerable reduction in complexity and energy consumption relative to the mechanical provision of flotation gas, the high flocculant dosage remains a major limitation of this method. High dosages of flocculant are required for effective flotation, which is not only costly but also deteriorates the downstream processes and the quality of the final product. Furthermore, a high level of flocculants in the culture medium may be toxic to microalgae, hampering continuing microalgae cultivation.

Therefore, there is a need for a method of cultivating microalgae which avoids the problems described above. In particular, there is a need for a method which avoids the use of complex and energy intensive equipment, and which avoids the use of flocculants.

### Summary of the Invention

It is one aim of the present invention, amongst others, to provide a method of cultivating microalgae that addresses at least one disadvantage of the prior art, whether identified here or elsewhere, or to provide an alternative to existing methods of microalgae cultivation. For instance, it may be an aim of the present invention to provide a method of cultivating microalgae which provides high-quality microalgae which may be harvested at low cost.

According to aspects of the present invention, there is provided a method of cultivating microalgae, cultivated microalgae, a use of such microalgae, a method of producing a bioproduct, and an apparatus as set forth in the appended claims. Other features of the invention will be apparent from the dependent claims, and the description which follows.

According to a first aspect of the present invention, there is provided a method of cultivating microalgae, the method comprising the steps of:
(a) providing a photoreactor;
(b) introducing a mixture of microalgae into the photoreactor;
(c) introducing a culture medium into the photoreactor;
(d) growing the microalgae in the culture medium in the photoreactor;
(e) separating the microalgae into a floating layer and a non-floating layer within the culture medium;
(f) optionally harvesting a portion of the microalgae in the floating layer; and
(g) discharging at least a portion of the non-floating layer from the photoreactor.

By "microalgae" we mean microscopic algae. Typically, microalgae are unicellular, but some may form filaments or colonies. Microalgae typically have a cell size ranging from about 1 µm to about 400 µm, for example from 2 to 200 µm.

Suitably the method according to the first aspect is a method of cultivating auto-floating microalgae.

The inventors have found that the method of the first aspect selects and cultivates auto-floating microalgae from a mixture of microalgae, resulting in enrichment of the auto-floating microalgae. The method advantageously does not require the use of flocculants or flotation gas, thereby greatly reducing the materials and energy required for cultivation and harvesting of the microalgae. The absence of flocculants leads to other advantages: it increases the quality of the microalgae ultimately obtained because the flocculants may otherwise interfere with the final application of the microalgae; it allows the culture medium to be reused for subsequent cultivation; and it reduces damage to the microalgal cells, allowing the healthy microalgal cells to be reused as an inoculum for subsequent cultivation. Another advantage of the method is that the enrichment of auto-floating microalgae is carried out in mixed culture conditions without needing to screen and isolate specific microalgal strains for inoculation and without need for closed reactors and sterile conditions. The steps of strain selection, cultivation and harvesting are integrated into a single process. This makes the method less labour intensive, as well as more suitable for large scale production.

By "auto-floating" we mean that the microalgae can naturally float at the surface of water without the need for chemical inducers (e.g. flocculants) or an external supply of flotation gas. It is believed that microalgae may exhibit auto-floatation activity through a variety of mechanisms, such as natural adhesion of micro oxygen gas bubbles resulting from photosynthesis to the cell surface; production of intracellular gas-filled structures (e.g. gas vacuoles or vesicles); production of liquid-filled floats; an increase in lipid content; and swimming. A variety of microalgal strains naturally found in the environment have auto-flotation activity. Typical auto-floating microalgae include (but are not limited to) green algae (Chlorophyceae) such as *Zygnemataceae, Oedogonium, Rhizoclonium, Microspora, Ulothrix, Spirogyra, Mougeotia, Zygnema, Cladophora, Hydrodictyon, Botryosphaerella* or *Chlorococcum;* yellow-green algae (Xanthophyceae) such as *Tribonema* or *Vaucheria;* diatoms (Baciallariophyceae) such as *Melosira, Aulacoseira, Cymbella* or *Gomphonema;* Cyanophyta such as *Spirulina, Microcystis, Aphanizomenon, Anabaena, Oscillatoria, Lyngbya, Phormidium, Plankthotrix* or *Nostoc;* flagellated organisms such as *Euglena.* Microalgae which are not auto-floating may be defined as non-floatable microalgae.

Steps (c) to (g) of the method according to the first aspect may collectively be referred to as a cycle. Steps (c) to (g) may be repeated as part of a cycle at least once, suitably at least 10 times, suitably at least 20 times, for example at least 50 times. Steps (a) and (b) are not repeated as part of the cycle, since a repeated cycle uses the photoreactor and the microalgae retained therein in the previous cycle. Each cycle may independently comprise or not comprise step (f). The number of cycles required for sufficient enrichment of auto-floating microalgae will depend on various conditions, such as which strains of microalgae are present in the initial mixture of microalgae.

The duration of a cycle of steps (c) to (g) may be referred to as the total cycle time. The total cycle time may be any suitable length of time, such as at least 12 hours, suitably from 1 to 30 days, suitably from 1 to 10 days, for example from 1 to 3 days . For example, the total cycle time may be 1 day. Alternatively, the total cycle time may be from 10 to 30 days, for example from 14 to 21 days. The total cycle time may vary from cycle to cycle. For example, the first cycle of steps (c) to (g) immediately following steps (a) and (b) may have a total cycle time which is longer than the total cycle time of subsequent cycles.

Step (a) of the method according to the first aspect comprises providing a photoreactor. The photoreactor may be any photoreactor suitable for the cultivation of microalgae. Typically, the photoreactor comprises a housing, wherein at least a portion of the housing may be transparent. The housing suitably defines an interior volume for containing a culture medium in which microalgae may be suspended. The photoreactor may be a closed photoreactor or an open photoreactor. The photoreactor is preferably a vertical photoreactor. Suitably the photoreactor is a column photoreactor or a flat-panel (or flat-plate) photoreactor.

In some embodiments step (a) comprises providing an apparatus according to the fifth aspect of the present invention as defined further below. The apparatus comprises a photoreactor.

Step (b) of the method according to the first aspect comprises introducing a mixture of microalgae into the photoreactor. The mixture of microalgae introduced into the photoreactor in step (b) may be referred to as an inoculum, and step (b) may be defined as an inoculation step. The mixture of microalgae (or inoculum) typically comprises two or more strains of microalgae. Suitably, the mixture of microalgae (or inoculum) comprises at least 5, preferably at least 10, for example at least 20 strains of microalgae. The mixture of microalgae (or inoculum) may comprise at least one strain of auto-floating microalgae. Alternatively, the mixture of microalgae (or inoculum) may not comprise auto-floating microalgae and at least one strain of auto-floating microalgae may be introduced into the photoreactor in a step other than step (b). For example, the auto-floating microalgae may be introduced via the culture medium and/or from the surrounding environment.

The mixture of microalgae may be provided as a mixture in water.

Suitably the mixture of microalgae comprises naturally occurring microalgae. By "naturally occurring" we mean that the microalgae occur in nature, and suitably have not been bred or genetically engineered. The mixture of microalgae may be obtained from one or more sources. Suitably the mixture of microalgae may be obtained from at least two, suitably at least three, for example at least four sources. Obtaining the mixture of microalgae from a variety of sources increases the likelihood that the mixture will comprise at least one strain of auto-floating microalgae, without the need to individually identify the strains of microalgae present in the mixture. Suitably at least a portion of the mixture of microalgae is obtained from the natural environment and/or wastewater. Preferably all of the mixture of microalgae is obtained from the natural environment and/or wastewater. By "obtained from the natural environment" we mean that the microalgae have not been cultured for use in the method according to the first aspect. Suitable sources of microalgae in the natural environment include ponds, lakes, rivers, and oceans. Examples of wastewater include domestic wastewater, industrial wastewater, agricultural wastewater and aquaculture wastewater.

Suitably, at least one strain of auto-floating microalgae is introduced into the photoreactor during the method of the first aspect. For example, the at least one strain of auto-floating microalgae may be introduced into the photoreactor via the mixture of microalgae in step (b), and/or via the culture medium in step (c). The culture medium introduced into the photoreactor in step (c) may comprise auto-floating microalgae, especially if the culture medium is not sterilised. The at least one strain of auto-floating microalgae may be introduced into the photoreactor during steps of the method other than steps (b) and (c), for example if the photoreactor is an open photoreactor.

Step (c) of the method according to the first aspect comprises introducing a culture medium into the photoreactor. The culture medium introduced into the photoreactor in step (c) may be referred to as fresh culture medium. Suitably, following step (b) and step (c), the microalgae are suspended in the culture medium.

The culture medium suitably comprises water and one or more nutrients, such as an aqueous solution of one or more nutrients. Any suitable nutrients may be used. Such nutrients are suitably in a form which is bioavailable to microalgae. Suitable nutrients for the growth of microalgae include nitrogen-containing nutrients, such as nitrates, ammonium and urea; phosphorus-containing nutrients, such as phosphates; carbon sources, such as carbon dioxide (CO₂), bicarbonates (HCO₃⁻) and organic sources of carbon; other macronutrients (such as S, K, Na, Fe, Mg and Ca) and micronutrients (such as B, Cu, Mn, Zn, Mo, Co, V and Se); and chelators (e.g. EDTA). The suitability of particular nutrients may depend on the strains of microalgae present in the mixture of microalgae. Suitable nitrogen-containing nutrients comprise nitrogen in a form which is bioavailable to microalgae and suitable phosphorus-containing nutrients comprise phosphorus in a form which is bioavailable to microalgae. The culture medium preferably comprises both nitrogen-containing nutrients and phosphorus-containing nutrients. The culture medium preferably also comprises other macro- and micronutrients, for example a carbon source.

The culture medium introduced into the photoreactor in step (c) (i.e. the fresh culture medium) suitably comprises one or more nitrogen-containing nutrients in an amount which provides at least 1 mg/L, preferably at least 5 mg/L, for example at least 10 mg/L of nitrogen. The culture medium may comprise one or more nitrogen-containing nutrients in an amount which provides from 1 to 1000 mg/L, preferably from 5 to 250 mg/L, for example from 10 to 100 mg/L of nitrogen. By "mg/L of nitrogen" we mean the weight, in mg, of nitrogen atoms provided by the nutrient per litre of culture medium. The culture medium suitably comprises one or more phosphorus-containing nutrients in an amount which provides at least 0.1 mg/L, preferably at least 0.5 mg/L, for example at least 1 mg/L of phosphorus. The culture medium may comprise one or more phosphorus-containing nutrients in an amount which provides from 0.1 to 100 mg/L, preferably from 0.5 to 30 mg/L, for example from 1 to 10 mg/L of phosphorus. By "mg/L of phosphorus" we mean the weight, in mg, of phosphorus atoms provided by the nutrient per litre of culture medium.

The culture medium may comprise an algal culture medium. An algal culture medium typically comprises a mixture of nutrients at a concentration suitable for the cultivation of microalgae. Examples of suitable algal culture media include BG11, BBM, COMBO, Kuhl, ES, ASW, and f/2 (details of which are well known to persons skilled in the art).

The culture medium may comprise or be prepared from nutrient-containing water, such as surface water, groundwater, runoff water, and wastewater. Examples of suitable wastewater include domestic wastewater, municipal wastewater, industrial wastewater, agricultural wastewater, and aquaculture wastewater. Wastewater is preferably pre-treated prior to use for the preparation of a culture medium. Such pre-treatment typically comprises removing organic matter, colour, solids, and turbidity. Sources of wastewater which have been pre-treated, such as secondary treated municipal wastewater, are especially suitable for the preparation of the culture medium. In some embodiments the culture medium is prepared from water obtained from natural water sources such as ponds, lakes, rivers and oceans. Suitably the culture medium is not sterilised.

The fresh culture medium may have a pH value of from 4 to 11. Preferably the fresh culture medium has a pH value of from 6 to 9.

Suitably the fresh culture medium is substantially free of flocculants. By "substantially free of flocculants" we mean that flocculants are not intentionally added to the culture medium, and if flocculants are present in the culture medium, they are only present in trace amounts (i.e. less than 0.1 wt%, preferably less than 0.01 wt% based on the weight of the culture medium). Preferably the fresh culture medium is completely free of flocculants. The method suitably does not comprise adding a flocculant to the microalgae in the culture medium. Preferably the fresh culture medium is substantially free of flocculants and the method does not comprise adding a flocculant to the culture medium.

By "flocculant" we mean any solid or liquid substance which may be added to a suspension of microalgae to help the microalgae to float, with or without the use of a flotation gas. Typically, a flocculant functions to aggregate microalgal cells. Examples of flocculants include aluminium sulphate, aluminium potassium sulphate, iron (III) hydroxide, polyaluminium chloride, and cationic polymers.

The culture medium may be introduced into the photoreactor by any suitable method, for example by pumping or pouring the culture medium into the photoreactor. The culture medium is preferably introduced into the photoreactor by pumping.

The volume of culture medium introduced into the photoreactor in step (c) may be selected to achieve a desired hydraulic retention time (HRT). The hydraulic retention time is defined herein as the ratio of the volume of the photoreactor to the volume of culture medium introduced into the photoreactor per day. The hydraulic retention time is suitably from 0.5 to 10 days, preferably from 1 to 5 days, for example from 1.4 to 3 days.

The duration of step (c) may be any suitable length of time, such as from 5 to 120 minutes, suitably from 10 to 60 minutes, for example from 20 to 50 minutes.

Step (b) and step (c) may be carried out in any suitable order. In one embodiment step (b) is followed by step (c). For example, a sample comprising a mixture of microalgae is introduced into the photoreactor, then a culture medium is introduced into the photoreactor such that the microalgae are suspended in the culture medium. In one embodiment step (b) and step (c) are carried out at least partially simultaneously, for example completely simultaneously. Steps (b) and (c) may be carried out simultaneously when the culture medium is present in the mixture of microalgae. For example, a mixture of microalgae may be suspended in a culture medium which is then introduced into the photoreactor, or a mixture of microalgae and a culture medium may be simultaneously independently introduced into the photoreactor. In another embodiment, step (c) is followed by step (b). For example, a culture medium may be introduced into the photoreactor, then a mixture of microalgae may be introduced into the photoreactor such that the microalgae are suspended in the culture medium.

In repeated cycles of steps (c) to (g), step (g) is suitably followed by step (c), followed by step (d). The volume of culture medium introduced into the photoreactor in step (c) of a repeated cycle is suitably substantially the same as the volume of culture medium in the non-floating layer discharged from the photoreactor in step (g) of a preceding cycle, for example step (g) of an immediately preceding cycle. This provides a substantially constant volume of culture medium for step (d) in each cycle.

Step (d) of the method according to the first aspect comprises growing the microalgae in the culture medium in the photoreactor. Step (d) may further comprise agitating the microalgae in the culture medium, i.e. as the microalgae grow.

Step (d) suitably comprises exposing the microalgae to light. The light may have a photosynthetic photon flux density (PPFD) of from 50 to 2000 µmol/m²/s, suitably from 75 to 1000 µmol/m²/S, for example from 100 to 400 µmolm²/s. For example, the light may have a PPFD of from 200 to 500 µmol/m²/s, suitably from 250 to 450 µmol/m²/S, for example from 300 to 400 µmol/m²/s. The PPFD is a measure of the intensity of photosynthetically active light (i.e. light having a wavelength of from 400 to 700 nm) and may be measured by a quantum meter.

Suitably the light comprises visible light, preferably light within the photosynthetic active spectrum (i.e. light having a wavelength of from 400 to 700 nm). The light may comprise natural light and/or artificial light, suitably natural light. By natural light we mean light from the sun. The use of natural light has the advantage of reducing capital and operating costs.

The microalgae may be exposed to light continuously or intermittently. The microalgae may be exposed to light for from 10 to 100%, suitably from 20 to 80%, for example from 30 to 70% of the duration of step (d). The microalgae may be exposed to light for from 6 to 24 hours per day, suitably from 8 to 20 hours per day, for example from 10 to 18 hours per day. The period during which the microalgae are exposed to light may be referred to as the photoperiod.

Step (d) may comprise supplying a carbon source to the microalgae in the culture medium. The carbon source functions as a nutrient to support the growth of the microalgae. The carbon source may comprise an inorganic carbon source and/or an organic carbon source. Preferably the carbon source comprises an inorganic carbon source. Suitable examples of inorganic carbon sources include carbonates (e.g. sodium carbonate and potassium carbonate), bicarbonates (e.g. sodium bicarbonate and potassium bicarbonate), and carbon dioxide. Preferably the carbon source comprises carbon dioxide. In some embodiments the carbon source is supplied as carbon dioxide in one or more of air, flue gas, biogas, or pure carbon dioxide gas, preferably in flue gas.

The amount of the carbon source supplied to the microalgae in the culture medium will typically be selected to maximise productivity of the microalgae, in particular to maximise the growth rate of the microalgae. The carbon source may be supplied to the microalgae in the culture medium in one or more discrete quantities, or continuously. The carbon source may be supplied to the microalgae in the culture medium while the microalgae are being exposed to light (i.e. during a light phase), or while the microalgae are not being exposed to light (i.e. during a dark phase). When the carbon source comprises carbon dioxide, it may be supplied in a concentration from 0.04% to 100%, suitably from 0.5% to 30%, preferably from 1% to 5% by volume. The carbon dioxide may be supplied continuously to the microalgae in the culture medium at a rate of from 0.005 to 10 vvm (gas volume flow per unit of liquid volume per minute), suitably from 0.05 to 5 vvm, for example from 0.1 to 3 vvm, depending on the concentration of carbon dioxide.

During step (d), the temperature of the culture medium/microalgae mixture is suitably maintained at a temperature which maximises growth of the microalgae. The temperature of the culture medium may be from 10 to 40°C, suitably from 10 to 30°C, preferably from 15 to 25°C.

Step (d) suitably comprises growing the microalgae in the culture medium under agitation. The agitation enhances the growth of the microalgae. Step (d) may comprise agitating the microalgae in the culture medium by any suitable means, such as by a mechanical means or by bubbling a gas through the culture medium (e.g. gas sparging). Suitable mechanical means include mechanical agitation devices, such as an impeller. Suitably the gas used to agitate the culture medium comprises a carbon source, such as carbon dioxide.

The first step (d) following step (b) may be referred to as a preculture step. The first step (d) following step (b) may be carried out until the culture medium comprises from 5 to 10 g/L by dry weight of microalgae. The first step (d) following step (b) may have a duration of from 1 to 3 weeks.

The dry weight of microalgae is suitably measured by filtering the microalgae through a 0.45 µm microfilter and oven drying the microalgae at 105°C for at least 2 hours.

Steps (c) and (d) may be carried out at least partially simultaneously. For example, the microalgae in the photoreactor may be exposed to light while a culture medium is being introduced into the photoreactor.

Step (e) of the method according to the first aspect comprises separating the microalgae into a floating layer and a non-floating layer within the culture medium. The non-floating layer may alternatively be referred to as a subnatant. Any suitable method of separation may be used.

Separating the microalgae suitably comprises keeping the culture medium comprising the microalgae static. By this we mean that the microalgae in the culture medium are not agitated during separation.

When step (d) comprises agitating the microalgae in the culture medium, step (e) may comprise stopping the agitation and allowing the microalgae to separate into the floating layer and the non-floating layer within the culture medium.

Step (e) may comprise vigorously agitating the microalgae in the culture medium in order to produce a turbulent liquid flow, and then stopping the agitation and allowing the microalgae to separate into the floating layer and the non-floating layer within the culture medium. Vigorous agitation of the microalgae in the culture medium may increase the separation efficiency of step (e).

Vigorous agitation of the microalgae may be achieved by any suitable means, such as a mechanical means. Suitable mechanical means include mechanical mixing devices, such as an impeller or a holding tank. For example, the culture medium containing the microalgae may be discharged into a holding tank, and reintroduced into the photoreactor within up to 5 minutes, suitably up to 3 minutes, preferably up to 1 minute, so as to create a vigorous flow.

After the agitation is stopped, the microalgae are suitably allowed to separate without further agitation.

Step (e) suitably comprises separating the microalgae (i.e. into a floating layer and a non-floating layer within the culture medium) for any suitable length of time, such as at least 0.1 hours, preferably at least 0.25 hours, for example at least 0.5 hours. Step (e) may comprise allowing the microalgae to separate for at least 0.1 hours, such as from 0.1 to 3 hours, suitably from 0.25 to 2 hours, for example from 0.5 to 1 hour. The time for which the microalgae are allowed to separate may be referred to as the separation time.

Step (e) may comprise exposing the microalgae to light during separation of the microalgae. The light may have a photosynthetic photon flux density (PPFD) of from 50 to 2000 µmol/m²/s, suitably from 75 to 1000 µmol/m²/s, for example from 100 to 400 µmolm²/s. For example, the light may have a PPFD of from 200 to 500 µmol/m²/S, suitably from 250 to 450 µmol/m²/S, for example from 300 to 400 µmol/m²/s.

Suitably the light comprises visible light, preferably light within the photosynthetic active spectrum (i.e. light having a wavelength of from 400 to 700 nm). The light may comprise natural light and/or artificial light, suitably natural light. The light in step (e) is suitably from the same source as the light in step (d).

Step (e) suitably has a separation efficiency of at least 50%, suitably at least 75%, for example at least 90%. The separation efficiency (%) is defined as 100 x (1-SS_{eff}/SSᵣ), where SS_{eff} is the microalgae concentration by dry weight (mg/L) in the non-floating layer and SSᵣ is the microalgae concentration by dry weight (mg/L) in the culture medium in the photoreactor prior to the separation.

Suitably step (e) does not comprise supplying a flotation gas to the microalgae in the culture medium. Suitably step (e) does not comprise carrying out dissolved air flotation, electrolytic flotation or dispersed air flotation on the microalgae in the culture medium. Preferably the method according to the first aspect does not comprise supplying a flotation gas to the microalgae in the culture medium. By a flotation gas, we mean a gas which is supplied as very fine bubbles to the microalgae in the culture medium. A flotation gas is typically generated by an electrochemical means or a mechanical means, such as by a sudden release of pressurised gas into the culture medium containing the microalgae.

The floating layer and the non-floating layer each comprise a portion of the microalgae in the culture medium in the photoreactor. Suitably, the floating layer and the non-floating layer together comprise all of the microalgae in the culture medium in the photoreactor. The floating layer is typically situated above the non-floating layer, for example on top of the non-floating layer in the photoreactor. The floating layer may be visually distinguished from the non-floating layer by the presence of floes of microalgae in the floating layer and the absence of floes of microalgae in the non-floating layer. The floating layer may comprise the highest 5 to 50%, suitably the highest 10 to 30%, for example the highest 20% by volume of the culture medium containing the microalgae in the photoreactor.

Suitably the microalgae in the floating layer comprise auto-floating microalgae, and the microalgae in the non-floating layer comprise non-floatable microalgae. Suitably the microalgae in the floating layer comprise a higher proportion by dry weight of auto-floating microalgae than the microalgae in the non-floating layer. Suitably the microalgae in the floating layer comprise a higher proportion by dry weight of auto-floating microalgae than the mixture of microalgae introduced into the photoreactor in step (b). A "higher proportion by dry weight" is suitably defined as the proportion by dry weight of auto-floating microalgae in the microalgae of the floating layer being at least 1.1 times, suitably at least 1.5 times, such as at least 2 times the proportion by dry weight of auto-floating microalgae in the microalgae of the non-floating layer or the mixture of microalgae introduced into the photoreactor in step (b).

Suitably step (d) is followed by step (e).

Step (f) of the method according to the first aspect comprises optionally harvesting a portion of the microalgae in the floating layer. When steps (c) to (g) are repeated in a cycle, each cycle may independently comprise or not comprise step (f).

Suitably step (f) comprises harvesting from 5 to 90%, suitably from 10 to 80%, for example from 20 to 50% by dry weight of the microalgae in the floating layer.

The amount of microalgae harvested may be selected to achieve a desired cell retention time (CRT). The cell retention time is defined herein as the ratio of the total dry weight of microalgae in the photoreactor (prior to harvesting) to the dry weight of microalgae harvested per day. The cell retention time may be selected to maximise microalgae productivity, to fine-tune the cellular composition of the microalgae, and to maintain a desired concentration of microalgae in the culture medium in the photoreactor. The cell retention time is suitably selected to achieve a concentration of microalgae in the culture medium of from 0.3 to 5 g/L, for example from 0.5 to 3 g/L, preferably from 1 to 2 g/L by dry weight of microalgae. The cell retention time is suitably from 1.25 to 30 days, preferably from 1.5 to 20 days, for example from 2 to 10 days.

Step (f) may comprise harvesting a portion of the microalgae in the floating layer by any suitable method, for example by use of a collecting device arranged within the photoreactor.

Suitably step (f) is only (optionally) carried out if there is at least 0.5 g/L, suitably at least 1 g/L by dry weight of microalgae in the culture medium in the photoreactor. Suitably step (f) is only (optionally) carried out if the separation efficiency of the preceding step (e) is at least 50%, suitably at least 75%, for example at least 90%. For the avoidance of doubt, when step (f) is not carried out because, for example, there is less than 0.5 g/L by dry weight of microalgae in the culture medium in the photoreactor, then step (f) is omitted.

The microalgae harvested in step (f) suitably comprise at least 50%, suitably at least 75%, for example at least 90% by dry weight of auto-floating microalgae.

Step (f), if present, suitably follows step (e). Suitably step (e) is followed by step (f), followed by step (g). Alternatively, step (e) is followed by step (g), followed by step (f). In repeated cycles of steps (c) to (g) where step (f) follows step (g), step (f) is followed by step (c), followed by step (d).

When step (f) is omitted, step (e) is suitably followed by step (g).

Step (g) of the method according to the first aspect comprises discharging at least a portion of the non-floating layer from the photoreactor.

Step (g) may comprise discharging the lowest 10 to 90%, suitably the lowest 30 to 80%, for example the lowest 50 to 70% by volume of the culture medium containing the microalgae in the photoreactor. Suitably step (g) comprises discharging the non-floating layer from the bottom of the photoreactor. This avoids accidentally discharging the floating layer from the photoreactor.

The culture medium containing the microalgae which is discharged from the photoreactor may be referred to as spent culture medium. The spent culture medium may be discarded.

Step (g) may comprise discharging at least 30%, suitably at least 50%, suitably at least 70%, for example at least 90% by volume of the non-floating layer from the photoreactor. Step (g) may comprise discharging from 10 to 90%, suitably from 30 to 80%, for example from 50 to 70% by volume of the culture medium containing the microalgae from the photoreactor.

The duration of step (g) may be any suitable length of time, such as from 5 to 120 minutes, suitably from 20 to 90 minutes, for example from 30 to 60 minutes.

Step (g) suitably comprises retaining at least a portion of the microalgae in the culture medium in the photoreactor. The microalgae retained in the photoreactor in step (g) typically comprise a mixture of microalgae. Step (g) suitably comprises retaining at least a portion of the floating layer in the photoreactor. Suitably all of the floating layer is retained in the photoreactor. Step (g) may comprise retaining at least a portion of the non-floating layer in the photoreactor, or may comprise discharging all of the non-floating layer from the photoreactor. When steps (c) to (g) are repeated as part of a cycle, the mixture of microalgae present in the photoreactor at the start of a new cycle is provided by the microalgae retained in the photoreactor at the end of a preceding cycle.

The steps of the method according to the first aspect may be carried out in any suitable order that provides the desired result of enrichment of auto-floating microalgae. The steps may be carried out in the order step (a); followed by step (b) and step (c); followed by step (d); followed by step (e); followed by step (f), if present, and step (g). The steps may be carried out in the order step (a); followed by step (b); followed by step (c); followed by step (d); followed by step (e); followed by step (f), if present, and step (g). The order of the steps may be varied as described above.

Suitably the method according to the first aspect comprises the steps of:
(a) providing a photoreactor;
(b) introducing a mixture of naturally occurring microalgae into the photoreactor;
(c) introducing a culture medium into the photoreactor, wherein the culture medium comprises an aqueous solution of one or more nitrogen-containing nutrients in an amount which provides at least 5 mg/L of nitrogen, and/or one or more phosphorus-containing nutrients which provides at least 0.1 mg/L of phosphorus;
(d) growing the microalgae in the culture medium in the photoreactor by exposing the microalgae to light and supplying a carbon source to the microalgae in the culture medium, and agitating the microalgae in the culture medium;
(e) stopping the agitation and allowing the microalgae to separate into a floating layer and a non-floating layer within the culture medium, wherein the microalgae are allowed to separate for at least 0.1 hours;
(f) optionally harvesting a portion of the microalgae in the floating layer if there is at least 0.5 g/L by dry weight of microalgae in the culture medium in the photoreactor;
(g) discharging at least 30% by volume of the non-floating layer from the photoreactor;
and repeating steps (c) to (g) as part of a cycle at least once,
wherein suitably at least one strain of auto-floating microalgae is introduced into the photoreactor during the method.

Suitably the method according to the first aspect comprises the steps of:
(a) providing a photoreactor;
(b) introducing a mixture of naturally occurring microalgae into the photoreactor, wherein the mixture of microalgae comprises at least 10 strains of microalgae;
(c) introducing a culture medium into the photoreactor, wherein the culture medium comprises an aqueous solution of one or more nitrogen-containing nutrients in an amount which provides at least 5 mg/L of nitrogen, and/or one or more phosphorus-containing nutrients which provides at least 0.5 mg/L of phosphorus, and wherein the culture medium does not comprise a flocculant;
(d) growing the microalgae in the culture medium in the photoreactor by exposing the microalgae to light and supplying carbon dioxide to the microalgae in the culture medium, and agitating the microalgae in the culture medium;
(e) vigorously agitating the microalgae in the culture medium and then stopping the agitation and allowing the microalgae to separate into a floating layer and a non-floating layer within the culture medium, wherein the microalgae are allowed to separate for at least 0.5 hours;
(f) optionally harvesting from 30 to 70 wt% of the microalgae in the floating layer if there is at least 1 g/L by dry weight of microalgae in the culture medium in the photoreactor;
(g) discharging at least 50% (for example at least 70%) by volume of the non-floating layer from the photoreactor;
and repeating steps (c) to (g) as part of a cycle at least 20 times,
wherein suitably at least one strain of auto-floating microalgae is introduced into the photoreactor during the method.

According to a second aspect of the present invention, there are provided microalgae cultivated in accordance with the method of the first aspect.

Such microalgae are suitably harvested via step (f) of the method of the first aspect.

The microalgae according to the second aspect suitably comprise at least 50 wt%, suitably at least 75 wt%, for example at least 90 wt% by dry weight of auto-floating microalgae.

Suitably the microalgae according to the second aspect are substantially free of flocculants. By "substantially free of flocculants" we mean that flocculants are not intentionally added to the microalgae, and if flocculants are present in the microalgae, they are only present in trace amounts (i.e. less than 0.1 wt%, preferably less than 0.01 wt% based on the dry weight of the microalgae). Preferably the microalgae are completely free of flocculants. Flocculants are as defined in relation to the first aspect.

Microalgae obtained by the method according to the first aspect have the advantage that they are free of flocculants, making the microalgae particularly suitable for further processing of the microalgae or for end applications of the microalgae such as animal or human consumption. Furthermore, the healthy microalgae cultivated by the method according to the first aspect are suitable for use as an inoculum for subsequent cultivation.

Microalgae typically contain various useful biomolecules including, but not limited to, carbohydrates, proteins and lipids. The cultivation conditions of the method according to the first aspect may be controlled to alter the content of biomolecules in the microalgae in view of the intended use of the microalgae.

The microalgae obtained by the method according to the first aspect may comprise from 40 to 75 wt%, suitably from 45 to 70 wt%, for example from 50 to 65 wt% of carbohydrates based on the dry weight of the microalgae. Microalgae rich in carbohydrates may be particularly suitable for the production of biomethane, bioethanol and biobutanol. Typically, the main component of the carbohydrates (e.g. more than 50 wt% of the carbohydrates) is starch or β-(1-3)/1-6)-glucans. β-(1-3)/1-6)-glucans have wide applications and may be suitable for the production of health products. Microalgae rich in carbohydrates may be prepared by exposing the microalgae in the culture medium in step (d) to light having a photosynthetic photon flux density of from 200 to 500 µmol/m²/s, suitably from 250 to 450 µmol/m²/s, for example from 300 to 400 µmolm²/s.

The microalgae obtained by the method according to the first aspect may comprise from 20 to 50 wt%, suitably from 25 to 45 wt%, for example from 30 to 40 wt% of lipids based on the dry weight of the microalgae. The microalgae may comprise from 5 to 40 wt%, suitably from 10 to 35 wt%, for example from 15 to 30 wt% of fatty acids (measured as fatty acid methyl esters, FAMEs) based on the dry weight of the microalgae. Microalgae rich in lipids, including FAMEs, may be particularly suitable for the production of biodiesel. Microalgae rich in lipids may be prepared by culturing the microalgae in accordance with the method of the first aspect, wherein the total cycle time is from 10 to 30 days, for example from 14 to 21 days.

According to a third aspect of the present invention, there is provided a use of microalgae according to the second aspect to produce a bioproduct, such as a biofuel or bio-based chemical.

According to a fourth aspect of the present invention, there is provided a method of producing a bioproduct, comprising the steps of:
(a) cultivating microalgae according to a method of the first aspect; and
(b) processing the microalgae to produce the bioproduct.

Bioproducts, and methods of processing microalgae to manufacture such bioproducts, will be known to a person skilled in the art.

Typically, processing the microalgae comprises dewatering the harvested microalgae. The harvested microalgae are suitably dewatered by a filter press or by centrifugation. The filter press may be under pressure or under a vacuum. Processing the microalgae may comprise dewatering the harvested microalgae to a solids content of at least 10%, suitably at least 15%, for example at least 20%. Dewatered microalgae are typically more suitable for transporting and for further processing into desirable products. Auto-floating microalgae typically have good dewatering properties.

The bioproduct may comprise a biofuel or a bio-based chemical. Biofuels may include, but are not limited to, biodiesel, bioethanol, biobutanol, biogas, biohydrogen, bio-oil, and syngas. Bio-based chemicals may include, but are not limited to, pigments, proteins, antioxidants, carotenoids, polysaccharides, fatty acids, and vitamins.

According to a fifth aspect of the present invention, there is provided a bioproduct produced in accordance with the method of the fourth aspect.

According to a sixth aspect of the present invention, there is provided an apparatus for cultivating microalgae, the apparatus comprising:
a photoreactor;
a means of supplying a carbon source to the photoreactor;
an agitation device; and
a collecting device.

The apparatus is preferably suitable for cultivating microalgae according to a method of the first aspect. The apparatus may be suitable for cultivating auto-floating microalgae.

The apparatus may be suitable for cultivating microalgae in batches and continuous cultivation.

The apparatus according to the sixth aspect comprises a photoreactor. The photoreactor may be any photoreactor suitable for the cultivation of microalgae. Typically, the photoreactor comprises a housing, wherein at least a portion of the housing may be transparent. The housing suitably defines an interior volume for containing a culture medium in which microalgae may be suspended. The photoreactor may be a closed photoreactor or an open photoreactor. The photoreactor is preferably a vertical photoreactor. Suitably the photoreactor is a column photoreactor or a flat-panel (or flat plate) photoreactor.

The photoreactor suitably comprises an inlet for receiving fresh culture medium and/or microalgae. The apparatus may comprise a feed pump for supplying fresh culture medium to the photoreactor via the inlet.

The apparatus may comprise a light source. The light source is suitably arranged such that the light source may illuminate microalgae suspended in culture medium in the photoreactor. The light source is suitably able to produce light having a photosynthetic photon flux density of from 50 to 2000 µmol/m²/s, suitably from 75 to 1000 µmol/m²/S, for example from 100 to 400 µmol/m²/s.

The means for supplying the carbon source may comprise a pump or a compressor for supplying the carbon source to the photoreactor. Where the carbon source is supplied by a gas, the means for supplying the carbon source suitably comprises a compressor, preferably a compressor in fluid communication with a diffusor. The diffusor is suitably arranged at the bottom of the photoreactor, such that the gas comprising the carbon source may rise through the culture medium contained in the photoreactor while in use. Where the carbon source is supplied by a liquid, the means for supplying the carbon source suitably comprises a pump. The photoreactor suitably comprises an inlet for receiving a carbon source.

The photoreactor suitably comprises an outlet for discharging spent culture medium. Suitably the outlet is arranged in the bottom 50%, preferably the bottom 25%, for example the bottom 10% by volume of the photoreactor. This prevents the outlet from discharging microalgae floating at the top of the culture medium when the apparatus is in use. The apparatus may comprise a discharge pump for removing spent culture medium from the photoreactor.

Suitably the photoreactor comprises an inlet for receiving fresh culture medium and an outlet for discharging spent culture medium.

The apparatus according to the sixth aspect comprises an agitation device suitably adapted to agitate microalgae suspended in a culture medium contained in the photoreactor. The agitation device may be any suitable agitation device, for example a mechanical agitation device such as an impeller. The agitation device is suitably arranged within the interior volume of the housing of the photoreactor. The apparatus may comprise a motor for driving the agitation device.

The apparatus according to the sixth aspect comprises a collecting device suitably adapted to collect microalgae floating in a culture medium from the photoreactor. Suitably the collecting device is arranged in the top 50%, preferably the top 25%, for example the top 10% by volume of the photoreactor. The collecting device may comprise any suitable collecting device, for example a scraper, a skimmer, a pump, an electromagnetic valve, or a combination thereof.

In summary, a method for cultivating microalgae is disclosed which comprises the steps of introducing a mixture of microalgae and a culture medium into a photoreactor, growing the microalgae, allowing the microalgae to separate into a floating layer and a non-floating layer within the culture medium, and discharging at least a portion of the non-floating layer from photoreactor. The method advantageously does not require the use of flocculants or flotation gas, reducing the energy and materials required, as well as increasing the quality of the microalgae obtained. Further, the method does not require screening and isolation of specific microalgal strains. Microalgae cultivated according to the method, a use of such microalgae to produce a bioproduct, a method of producing a bioproduct, a bioproduct produced by such a method, and an apparatus for cultivating microalgae are also described.

### Brief Description of the Drawings

For a better understanding of the invention, and to show how example embodiments may be carried into effect, reference will now be made to the accompanying drawings in which:
Figure 1 is a schematic diagram of an apparatus according to the sixth aspect of the present invention.
Figure 2 shows a typical operating sequence in a method according to the first aspect of the present invention.
Figure 3 is a graph showing the evolution of microalgae concentration and separation efficiency in Example 1 overtime.

### Detailed Description of the Example Embodiments

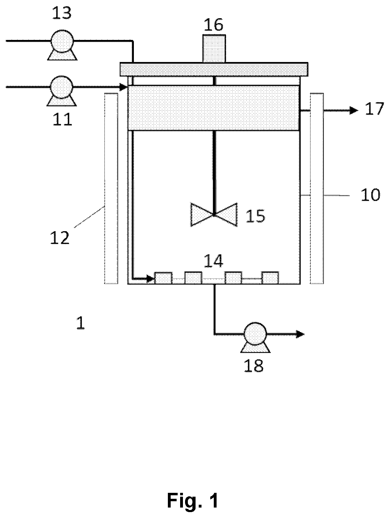

Figure 1 shows an apparatus (1) according to the sixth aspect of the present invention, comprising a photoreactor (10). Fresh culture medium and a mixture of microalgae are introduced to the photoreactor (10) by a feed pump (11). The microalgae suspended in a culture medium inside the photoreactor (10) are grown by providing them with illumination from a light source (12) arranged adjacent to the photoreactor (10), and agitating them by means of an impeller (15) driven by a motor (16). A carbon source is supplied to the growing microalgae by means of a compressor (13), which compresses carbon dioxide-containing gas, and a diffusor (14) which disperses the compressed carbon dioxide-containing gas into the culture medium in which the microalgae are suspended. Once the microalgae suspended in the culture medium in the photoreactor (10) have been grown, they are allowed to separate into a floating layer and a non-floating layer within the culture medium by stopping agitation. A collecting device (17) is arranged such that it may harvest microalgae from the floating layer, which suitably comprises auto-floating microalgae. After separation of the microalgae into a floating layer and a non-floating layer, and optional harvesting of microalgae from the floating layer, spent culture medium, which suitably comprises non-floatable microalgae, is discharged from the bottom of the photoreactor (10) by means of a discharge pump (18).

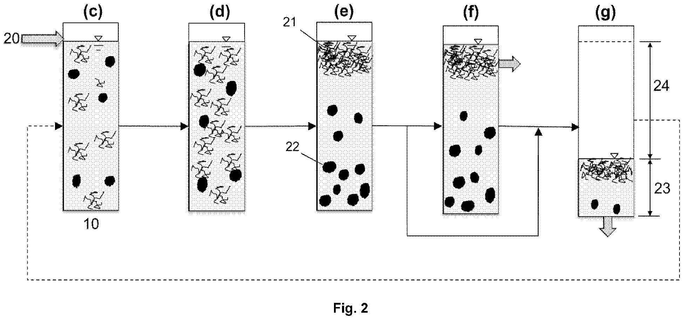

Figure 2 shows steps (c) to (g) of a method according to the first aspect of the present invention. Step (c) comprises feeding fresh culture medium (20) into a photoreactor (10), resulting in a suspension of a mixture of microalgae in the culture medium. The mixture of microalgae may have been introduced into the photoreactor in step (b) (not shown) or left over from a previous cycle of steps (c) to (g), and in this example comprises at least one strain of auto-floating microalgae and at least one strain of non-floatable microalgae. Step (d) comprises growing the microalgae in the culture medium in the photoreactor under agitation, causing the weight of microalgae in the culture medium in the photoreactor to increase. Suitably step (d) comprises exposing the microalgae to light and supplying a carbon source to the microalgae in the culture medium. Agitation is provided by means of a mechanical device or gas sparging. Step (e) comprises a short period of vigorous agitation followed by stopping the agitation and allowing the microalgae to separate into a floating layer (21) comprising auto-floating microalgae and a non-floating layer (22) comprising non-floatable microalgae within the culture medium. Microalgae may be harvested from the floating layer (21) in optional step (f), suitably by means of a collecting device. Whether step (f) is carried out may depend on the concentration of the microalgae in the culture medium in the photoreactor, or on the separation efficiency of step (e). Step (g) comprises discharging at least a portion of the non-floating layer (22). At least a portion (23) of the culture medium and microalgae is retained in the photoreactor, suitably comprising all of the floating layer (21) and a portion of the non-floating layer (22). The cycle of steps (c) to (g) may be repeated one or more times. The volume of fresh culture medium (20) fed into the photoreactor in a repeated step (c) is suitably the same as the volume of the culture medium (24) discharged in step (g).

### Examples

### Example 1

A laboratory-scale column photoreactor (volume=6.65 L) was constructed from a Plexiglas^{®} tube and used to cultivate microalgae under the following conditions:

| | |
|---|---|
| **Total cycle time (hours)** | 24 |
| **Photoperiod (hours/day)** | 10 |
| **Photosynthetic photon flux density (µmol/m²/s)** | 100-350 |
| **Inorganic carbon source** | 5% CO₂ |
| **Rate of inorganic carbon supply (mL/minute)** | 30-60 |
| **Hydraulic retention time (days)** | 1.4 |
| **Cell retention time (days)** | 5 |
| **Culture pH** | 6-9 |
| **Temperature (°C)** | 16-25 |
| **Level of NO₃⁻-N (mg/L)** | 10 |
| **Level of PO₄³⁻-P (mg/L)** | 1 |

Secondary effluent from a local wastewater treatment plant in Galway, Ireland was used as the culture medium.

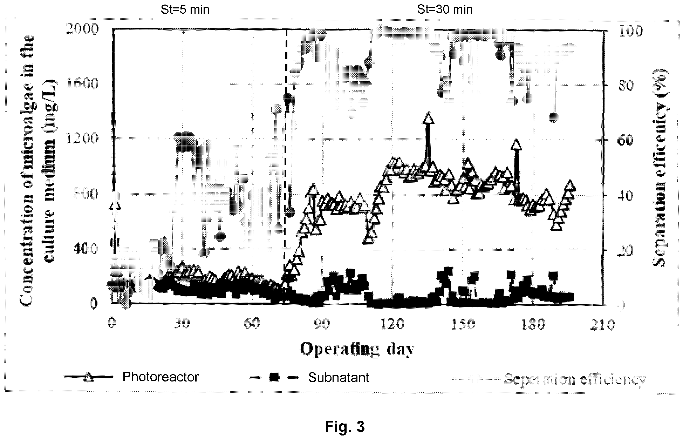

At the start-up, a highly diverse mixed algal sample (containing more than 20 species) sourced from wastewater (digestate, secondary effluent) and a local river was used as inoculum. The continuous laboratory test started with a separation time (St) of 5 minutes and a photosynthetic photon flux density of 100 µmol/m²/s for 75 days. Prior to the separation of the microalgae in the culture medium, turbulent liquid flow was created manually by discharging all the mixed liquor into a holding tank, followed by refilling the mixed liquor into the reactor in a short period of time (about 1 minute). During this period, some auto-floating microalgae appeared, but the culture was still dominated by non-floatable microalgae with inadequate separation efficiency (Fig. 3).

The separation time was extended to 30 minutes from day 76, after which a highly productive auto-floating microalga (*Tribonema* sp., Xanthophyta) rapidly became dominant and the separation efficiency increased remarkably to >90% within one week (Figure 3). Thereafter, the separation efficiency remained above 80% for most of the time, with an average of 91 %. The harvested microalgae had excellent dewatering characteristics with specific resistance to filtration values below 10¹⁰ m/kg, which facilitated economical dewatering of the harvested microalgae. The microalgae were easily dewatered with microfiltration to a solids content of 20-23%, without coagulant addition.

From day 87 the microalgae were exposed to a photosynthetic photon flux density of 350 µmo/m²/s. The microalgae accumulated a large amount of carbohydrates (52-62% of dry cell weight), which sustained complete nutrient removal during the dark phase. The main components of the carbohydrates were β-(1-3/1-6)-glucans (about 60 wt% of the total carbohydrates). For comparison, the carbohydrates content of the microalgae prior to day 87 was 28.8-32.5% of dry cell weight.

### Example 2

Example 1 was repeated, except that auto-floating microalgae obtained in Example 1 were used as inoculum, BG11 was used as the culture medium, and the total cycle time was set to 14-21 days. The microalgae accumulated a significant amount of lipids (30-40% by dry weight as total lipids; 15-30% by dry weight as FAMEs). For comparison, the microalgae obtained in Example 1 had a lipids content of around 10% by dry weight.

The example embodiment described above provides a method for cultivating microalgae without the use of flocculants or flotation gas, where the biochemical content of the microalgae obtained can be altered by tuning the method parameters.

The harvesting of microalgae by using flocculants creates problems regarding the cost of microalgae production and the quality of the microalgae obtained. These problems may be addressed by example embodiments as described herein.

Although a few preferred embodiments have been shown and described, it will be appreciated by those skilled in the art that various changes and modifications might be made without departing from the scope of the invention, as defined in the appended claims.

Throughout this specification, the term "comprising" or "comprises" means including the component(s) specified but not to the exclusion of the presence of other components. The term "consisting essentially of" or "consists essentially of" means including the components specified but excluding other components except for materials present as impurities, unavoidable materials present as a result of processes used to provide the components, and components added for a purpose other than achieving the technical effect of the invention. Typically, when referring to compositions, a composition consisting essentially of a set of components will comprise less than 5% by weight, typically less than 3% by weight, more typically less than 1% by weight of non-specified components.

The term "consisting of" or "consists of" means including the components specified but excluding addition of other components.

Whenever appropriate, depending upon the context, the use of the term "comprises" or "comprising" may also be taken to encompass or include the meaning "consists essentially of" or "consisting essentially of", and may also be taken to include the meaning "consists of" or "consisting of".

For the avoidance of doubt, wherein amounts of components in a composition are described in wt%, this means the weight percentage of the specified component in relation to the whole composition referred to. For example, "the mixture of microalgae comprises up to 20 wt% of auto-floating microalgae" means that up to 20 wt% of the mixture of microalgae is provided by auto-floating microalgae.

The optional features set out herein may be used either individually or in combination with each other where appropriate and particularly in the combinations as set out in the accompanying claims. The optional features for each aspect or exemplary embodiment of the invention as set out herein are also to be read as applicable to any other aspect or exemplary embodiments of the invention, where appropriate. In other words, the skilled person reading this specification should consider the optional features for each exemplary embodiment of the invention as interchangeable and combinable between different exemplary embodiments.

Attention is directed to all papers and documents which are filed concurrently with or previous to this specification in connection with this application and which are open to public inspection with this specification, and the contents of all such papers and documents are incorporated herein by reference.

All of the features disclosed in this specification (including any accompanying claims, and drawings), and/or all of the steps of any method or process so disclosed, may be combined in any combination, except combinations where at least some of such features and/or steps are mutually exclusive.

Each feature disclosed in this specification (including any accompanying claims, and drawings) may be replaced by alternative features serving the same, equivalent or similar purpose, unless expressly stated otherwise. Thus, unless expressly stated otherwise, each feature disclosed is one example only of a generic series of equivalent or similar features.

The invention is not restricted to the details of the foregoing embodiment(s). The invention extends to any novel one, or any novel combination, of the features disclosed in this specification (including any accompanying claims, and drawings), or to any novel one, or any novel combination, of the steps of any method or process so disclosed.

## Claims

1. A method for cultivating microalgae, the method comprising the steps of:
(a) providing a photoreactor;
(b) introducing a mixture of microalgae into the photoreactor;
(c) introducing a culture medium into the photoreactor;
(d) growing the microalgae in the culture medium in the photoreactor;
(e) separating the microalgae into a floating layer and a non-floating layer within the culture medium;
(f) optionally harvesting a portion of the microalgae in the floating layer; and
(g) discharging at least a portion of the non-floating layer from the photoreactor.

2. The method of claim 1 wherein steps (c) to (g) are repeated in a cycle at least once, preferably wherein the volume of culture medium introduced into the photoreactor in step (c) of a repeated cycle is substantially the same as the volume of culture medium in the non-floating layer discharged from the photoreactor in step (g) of a preceding cycle.

3. The method of any preceding claim wherein at least one strain of auto-floating microalgae is introduced into the photoreactor during the method and/or wherein the mixture of microalgae in step (b) comprises naturally occurring microalgae.

4. The method of any preceding claim wherein the microalgae in the floating layer in step (e) comprises a higher proportion by dry weight of auto-floating microalgae than the microalgae in the non-floating layer.

5. The method of any preceding claim wherein step (d) comprises exposing the microalgae to light, preferably natural light.

6. The method of any preceding claim wherein step (d) comprises supplying a carbon source to the microalgae in the culture medium, preferably wherein the carbon source comprises carbon dioxide.

7. The method of any preceding claim, wherein step (d) comprises agitating the microalgae in the culture medium, preferably wherein step (e) comprises stopping the agitation and allowing the microalgae to separate into the floating layer and the non-floating layer within the culture medium.

8. The method of any preceding claim wherein step (g) comprises discharging at least 30% by volume of the non-floating layer from the photoreactor.

9. The method of any preceding claim wherein the culture medium introduced into the photoreactor in step (c) is substantially free of flocculants and/or wherein the method does not comprise adding a flocculant to the microalgae in the culture medium.

10. The method of any preceding claim wherein step (e) does not comprise supplying a flotation gas to the microalgae in the culture medium.

11. Microalgae cultivated in accordance with the method of any preceding claim.

12. Use of the microalgae of claim 11 to produce a bioproduct, such as a biofuel or a bio-based chemical.

13. A method of producing a bioproduct, such as a biofuel or a bio-based chemical, the method comprising the steps of:
(a) cultivating microalgae according to the method of any of claims 1 to 10; and
(b) processing the microalgae to produce the bioproduct.

14. A bioproduct produced in accordance with the method of claim 13.

15. An apparatus for cultivating microalgae, the apparatus comprising:
a photoreactor;
a means of supplying a carbon source to the photoreactor;
an agitation device; and
a collecting device,
preferably wherein the photoreactor comprises an inlet for receiving fresh culture medium and an outlet for discharging spent culture medium.
